# EUROPEAN PATENT APPLICATION

(11) **EP 3 308 789 A1**
(43) Date of publication of application: **18.04.2018**
(21) Application number: 16807600.8
(22) Date of filing: 10.06.2016
(51) Int. Cl.: A61K 31/7088, A61K 9/127, A61K 47/24, A61K 48/00, A61P 43/00

(54) **NUCLEIC ACID DELIVERY CARRIER, NUCLEIC ACID DELIVERY KIT, AND NUCLEIC ACID DELIVERY METHOD**

(30) Priority: 10.06.2015 JP 2015117429
(71) Applicant: Keio University, Tokyo 108-8345 (JP)
(72) Inventor: KANAZAWA, Hideko, Tokyo 105-8512 (JP); WANG, Jian, Tokyo 105-8512 (JP)
(74) Representative: Appleyard Lees IP LLP
(86) International application number: PCT/JP2016/067382
(87) International publication number: WO 2016/199895

(57) **Abstract**

The purpose of the present invention is to provide a nucleic acid delivery carrier, a nucleic acid delivery kit, and a nucleic acid delivery method which have an excellent effect of inhibiting cell death after nucleic acid delivery and have excellent efficiency of delivering nucleic acids into cells. This carrier for delivery of nucleic acids into cells comprises a liposome having a membrane fusion capability, wherein the liposome includes a cationic lipid as a membrane constituent, and the surface of the liposome is modified with a temperature-responsive polymer. This nucleic acid delivery carrier is optimally used to deliver nucleic acids into cervical cancer cells. This kit for delivering nucleic acids into cells includes the aforementioned delivery carrier. The nucleic acid delivery method for delivering nucleic acids into cells involves a step for contacting isolated cells with a complex of the aforementioned nucleic acid delivery carrier and nucleic acids, or, a step for dosing a non-human animal with a complex of the aforementioned nucleic acid delivery carrier and nucleic acids.

## Description

### TECHNICAL FIELD

The present invention relates to a carrier for delivering a nucleic acid into a cell, a kit for delivering a nucleic acid, and a method of delivering a nucleic acid into a cell.

### BACKGROUND ART

In gene therapy, delivery of a nucleic acid such as siRNA into a target cell is difficult when the nucleic acid such as siRNA alone is administered intravenously. This is because the nucleic acid such as siRNA tends to disappear from the blood stream due to uptake by the reticuloendothelial system (RES) and decomposition by nuclease and the like. For this reason, there have always been demands for a carrier which is suitable for delivering a nucleic acid more efficiently into a target cell, and can be used for a drug delivery system (DDS).

Meanwhile, in recent years, use of a liposome for DDS has attracted much attention. A liposome is a spherical particle having a bimolecular lipid membrane structure similar to cell membrane, and is also used for DDS of a nucleic acid because it forms a complex with a nucleic acid to retain the nucleic acid stably.

For example, Lipofectamine® available from Invitrogen, Inc. (see Nonpatent Document 1) is traditionally used as a carrier for DDS which can efficiently deliver siRNA into cells.

Non-Patent Document 1: https://www.lifetechnologies.com/order/catalog/product/1377803 0

### DISCLOSURE OF THE INVENTION

### Problems to be Solved by the Invention

However, the above Lipofectamine® has a good delivery efficiency of a nucleic acid into a cell, but disadvantageously tends to cause cell death after delivery of the nucleic acid.

Cell death after delivery of a nucleic acid may be reduced when the surface of a liposome is modified with PEG (polyethylene glycol). However, a liposome having a surface modified with PEG shows a low delivery efficiency of a nucleic acid into a cell.

A carrier for delivering a nucleic acid has not yet been available to date which can reduce cell death after delivery of the nucleic acid, and show excellent delivery efficiency of a nucleic acid into a cell.

The present invention was made in view of the above actual circumstances. An object of the present invention is to provide a carrier for delivering a nucleic acid, a kit for delivering a nucleic acid, and a method of delivering a nucleic acid, which show an excellent effect for reducing cell death after delivery of a nucleic acid and excellent delivery efficiency of a nucleic acid into a cell.

### Means for Solving the Problems

The present inventors found that a liposome including a cationic lipid as a membrane constituent and having a surface modified with a temperature-responsive polymer can show excellent delivery efficiency of a nucleic acid into a cell while reducing cell death after delivery of the nucleic acid. Then the present invention has been completed. More specifically, the present invention can provide the followings.
(1) A carrier for delivering a nucleic acid into a cell including a liposome having a membrane fusion capability, in which
   the liposome includes a cationic lipid as a membrane constituent, and
   a surface of the liposome is modified with a temperature-responsive polymer.
(2) The carrier for delivering a nucleic acid according to (1), in which the temperature-responsive polymer has a lower critical solution temperature of 35.0 to 45.0°C with water.
(3) The carrier for delivering a nucleic acid according to (1) or (2), in which the liposome includes a phospholipid as a membrane constituent.
(4) The carrier for delivering a nucleic acid according to any one of (1) to (3), which is used for delivering the nucleic acid into a cervical cancer cell.
(5) A kit for delivering a nucleic acid into a cell, including the carrier for delivering a nucleic acid according to any one of (1) to (4).
(6) A method of delivering a nucleic acid into a cell, the method including the step of: contacting a complex of the carrier for delivering a nucleic acid according to any one of (1) to (4) and a nucleic acid with an isolated cell, or administering the complex of the carrier for delivering a nucleic acid and a nucleic acid to a non-human animal.

The present invention can provide a carrier for delivering a nucleic acid, a kit for delivering a nucleic acid, and a method of delivering a nucleic acid, which show an excellent effect for reducing cell death after delivery of a nucleic acid and excellent delivery efficiency of a nucleic acid into a cell.

### BRIEF DESCRIPTION OF THE DRAWINGS

Fig. 1 is a graph illustrating results from measurements of the luciferase activity for Example 1, Comparative Example 1, and Control Examples 1 and 2.
Fig. 2 is a graph illustrating results from measurements of the luciferase activity for Example 1, Comparative Examples 1 to 3, and Control Example 2.
Fig. 3 is a graph illustrating results from measurements of the cell viability for Example 1, Comparative Example 1, and Control Examples 3 and 4.
Fig. 4 is a graph illustrating results from measurements of the luciferase activity at 30°C or 40°C for Example 1, Comparative Example 1, and Comparative Example 3.
Fig. 5 is a graph illustrating results from measurements of the luciferase activity at 37°C or 42°C for Example 2.
Fig. 6 is a graph illustrating results from measurements of the fluorescence intensity of a liposome having carboxyfluorescein encapsulated according to Example 1 before and after treatment with Triton.
Fig. 7 is a graph illustrating results of measurements of the luciferase activity for a liposome having carboxyfluorescein encapsulated according to Example 1, and a liposome according to Comparative Example 1.
Fig. 8 is a graph illustrating the percentage of cells expressing GFP when a plasmid (the MSCV-IRES-GFP plasmid) was transfected into HeLa cells using Example 1, Comparative Example 1, and Comparative Example 3.

### PREFERRED MODE FOR CARRYING OUT THE INVENTION

Below, the embodiments of the present invention will be described, but the present invention shall not be limited to these. They can be suitably altered.

### <Career for delivering nucleic acid>

The carrier for delivering a nucleic acid according to an embodiment of the present invention is a carrier for delivering a nucleic acid into a cell including a liposome having a membrane fusion capability, in which the liposome includes a cationic lipid as a membrane constituent, and a surface of the liposome is modified with a temperature-responsive polymer. By virtue of having the above structure, the carrier for delivering a nucleic acid according to an embodiment of the present invention has an excellent effect for reducing cell death after delivery of the nucleic acid and excellent delivery efficiency of a nucleic acid into a cell.

The reasons why the carrier for delivering a nucleic acid according to an embodiment of the present invention has an excellent effect for reducing cell death after delivery of the nucleic acid and excellent delivery efficiency of a nucleic acid into a cell can be explained as follows.

The surface of the liposome of the carrier for delivering a nucleic acid according to an embodiment of the present invention is modified with a temperature-responsive polymer. This temperature-responsive polymer can form a hydration layer at or below the lower critical solution temperature to reduce cytotoxicity due to a cationic lipid. Further, when the temperature-responsive polymer becomes hydrophobic at an elevated temperature, the hydration layer formed is decreased, leading to improved cell affinity. This enables direct delivery of the nucleic acid into a cell without via a lysosome by virtue of a lipid having a membrane fusion capability. As a result, an excellent effect for reducing cell death after delivery of a nucleic acid and excellent delivery efficiency of a nucleic acid into a cell can be conferred on the carrier for delivering a nucleic acid according to an embodiment of the present invention.

There is no particular limitation for the lipid which constitutes the liposome according to an embodiment of the present invention as long as it has a membrane fusion capability and includes a cationic lipid as a membrane constituent. For example, it may be formed with a cationic lipid and a lipid or macromolecule having a membrane fusion capability, or the cationic lipid itself may have a membrane fusion capability. For the liposome according to an embodiment of the present invention, the lipid included as a membrane constituent may be of one type or a combination of two or more types. It is noted that the term "membrane fusion capability" as used in the present invention refers to a capability to fuse with an endosomal membrane or a cell membrane.

Cationic lipids include cationic lipids which are not a phospholipid, for example, 1,2-dioleoyl-3-trimethylammonium propane (DOTAP), N-[1-(2,3-dioleoylpropyl)]-N,N-dimethylamine (DODAP), N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTMA), N,N-dimethyl-2,3-dioleyloxypropylamine (DODMA), 2,3-dioleyloxy-N-[2-(sperminecarboxyamide)ethyl]-N,N-dimethyl-1-propanaminium trifluoroacetic acid (DOSPA), N-[1-(2,3-ditetradecyloxypropyl)]-N,N-dimethyl-N-hydroxyethylammonium bromide (DMRIE), N-[1-(2,3-dioleyloxypropyl)]-N,N-dimethyl-N-hydroxyethylammonium bromide (DORIE), and the like. Alternatively they include cationic phospholipids and the like. Among these, 1,2-dioleoyl-3-trimethylammonium propane (DOTAP), DODAP, DMRIE, and DORIE are preferably used in view of their excellent delivery efficiency of a nucleic acid into a cell. These may be used alone or in combinations of two or more.

Lipids which can be used as a membrane constituent along with a cationic lipid to confer a membrane fusion capability on the liposome according to an embodiment of the present invention (hereinafter, may be referred to as the "membrane fusogenic lipid" in the present specification) include, for example, phospholipids such as natural phospholipids, for example, phosphatidylethanolamine, phosphatidylcholine, lysophosphatidylcholine, phosphatidylglycerol, phosphatidic acid, phosphatidylserine, phosphatidylinositol, cardiolipin, sphingomyelin, soybean lecithin, yolk lecithin, lysolecithin, and the like; high molecular weight succinylated polyglycidol (SucPG) having a membrane fusion capability; and the like. Among these, phosphatidylethanolamine and succinylated polyglycidol are particularly preferred in view of their excellent delivery efficiency of a nucleic acid into a cell. Moreover, the liposome according to an embodiment of the present invention preferably includes a phospholipid as a membrane constituent in view of its ability of further reducing cell death due to a cationic lipid.

Examples of phosphatidylethanolamine include 1,2-dioleoyl-sn-glycero-3-phosphoethanolamine (DOPE), 1,2-dilinoleoyl-sn-glycero-3-phosphoethanolamine (DLoPE), 1,2-dierucoyl-sn-glycero-3-phosphoethanolamine (DEPE), 1,2-dimyristoyl-sn-glycero-3-phosphoethanolamine (DMPE), 1,2-dipalmitoyl-sn-glycero-3-phosphoethanolamine (DPPE), 1,2-distearoyl-sn-glycero-3-phosphoethanolamine (DSPE), and the like. Among these, 1,2-dioleoyl-sn-glycero-3-phosphoethanolamine (DOPE), 1,2-dipalmitoyl-sn-glycero-3-phosphoethanolamine (DPPE) is particularly preferred in view of their excellent delivery efficiency of a nucleic acid into a cell.

In view of ability of further reducing cell death after delivery of a nucleic acid and excellent delivery efficiency of a nucleic acid into a cell, the liposome according to an embodiment of the present invention preferably includes a cationic lipid that is not a phospholipid and a membrane fusogenic lipid as membrane constituents in a molecular ratio suitable for achieving excellent efficiency for delivering a nucleic acid into a cell, preferably in a molecular ratio of 3:0.5 to 3:20, more preferably in a molecular ratio of 3:2 to 3:15, and even more preferably in a molecular ratio of 3:5 to 3:10.

Further, 1,2-dioleoyl-3-trimethylammonium propane (DOTAP) as a cationic lipid and 1,2-dioleoyl-sn-glycero-3-phosphoethanolamine (DOPE) as a membrane fusogenic lipid are in particular preferably included in the above molecular ratios as membrane constituents of a liposome. Moreover, when the surface of a liposome surface is modified with a temperature-responsive polymer modified with a lipid as a membrane constituent of the liposome, the temperature-responsive polymer modified with the lipid is preferably present in an amount of 0.05 to 40 mol% relative to the entire lipids as membrane constituents, more preferably 1.0 to 20 mol%, and even more preferably 2.0 to 8.0 mol% in view of ability of further reducing cell death after delivery of a nucleic acid and excellent delivery efficiency of a nucleic acid into a cell. Furthermore, the temperature-responsive polymer is preferably modified with a membrane fusogenic lipid, and in particular preferably modified with 1,2-dioleoyl-sn-glycero-3-phosphoethanolamine (DOPE) among membrane fusogenic lipids.

The temperature-responsive polymer forms a hydration layer over the surface of a liposome at or below the lower critical solution temperature (LCST) with water, reducing the affinity of a liposome for a cell membrane whilst it aggregates and becomes hydrophobic at a temperature at or above the lower critical solution temperature, improving the affinity of the liposome for the cell membrane. As described above, if a temperature-responsive polymer for use in an embodiment of the present invention has a low lower critical solution temperature with water, the affinity of a liposome for a cell membrane is reduced, resulting in decreased delivery efficiency into cells. Therefore, the lower critical solution temperature with water of a temperature-responsive polymer for use in an embodiment of the present invention is preferably at a temperature around the body temperature (for example, 35.0 to 45.0°C). On the other hand, a temperature-responsive polymer for use in an embodiment of the present invention having a lower critical solution temperature as high as possible within a temperature range around the body temperature tends to more easily reduce cell death after delivery of a nucleic acid into a cell. Therefore, the temperature-responsive polymer preferably has a lower critical solution temperature of preferably 37.0 to 42.0°C with water, more preferably 37.5 to 41.0°C, and even more preferably 38.0 to 40.5°C in view of excellent delivery efficiency of a nucleic acid into a cell and ability of more easily reducing cell death after delivery of a nucleic acid into a cell. It is noted that in the present invention, the lower critical solution temperature of a temperature-responsive polymer with water can be measured by differential scanning calorimetry (DSC) .

Specific examples of the temperature-responsive polymer for modifying the surface of the liposome according to an embodiment of the present invention may include, for example, a polymer having a (meth)acrylamide compound, an N-(or N,N-di)alkyl-substituted (meth)acrylamide derivative, a vinyl ether derivative, or the like as a constituent monomer, or a copolymer having two or more of these monomers. Further, a copolymer of monomers other than these monomers, a substance obtainable by graft polymerization or copolymerization of polymers, or a mixture of a homopolymer and copolymer of these monomers may be used. Moreover, the temperature-responsive polymer may be cross-linked as long as the original properties of the polymer are not impaired.

Specific monomers for constituting a temperature-responsive polymer include, for example, N-isopropyl(meth)acrylamide, N-n-propyl(meth)acrylamide, N-cyclopropyl(meth)acrylamide, N-ethoxyethyl(meth)acrylamide, N-tetrahydrofurfuryl(meth)acrylamide, N,N-ethylmethyl(meth)acrylamide, N,N-diethyl(meth)acrylamide, and the like. The temperature-responsive polymer may be a homopolymer of these monomers, or a copolymer of two or more. Further, monomers which may be further co-polymerized with these monomers include dimethylaminopropyl(meth)acrylamide, (meth)acrylamide, dimethylamino(meth)acrylamide, N,N-diethyl(meth)acrylamide, N,N-dimethyl(meth)acrylamide, ethylene oxide, (meth)acrylic acid and salts thereof, hydroxyethyl(meth)acrylate, polyhydroxyethyl(meth)acrylate, vinyl alcohol, vinyl pyrrolidone, cellulose, carboxymethylcellulose, and the like. The lower critical solution temperature of a temperature-responsive polymer with water can be adjusted by appropriately selecting a monomer for constituting the temperature-responsive polymer among those described above according to their properties.

A compound represented by the following general formula (1) as a copolymer of N-isopropyl(meth)acrylamide (NIPAA) and dimethylaminopropylacrylamide (DMAPAA) is preferably used as a temperature-responsive polymer in view of excellent delivery of a nucleic acid into a cell and ability of more easily reducing cell death after delivery of a nucleic acid into a cell. In the following formula (1), x is preferably 60 to 99, more preferably 90 to 99, and y is preferably 1 to 40, more preferably 1 to 10.

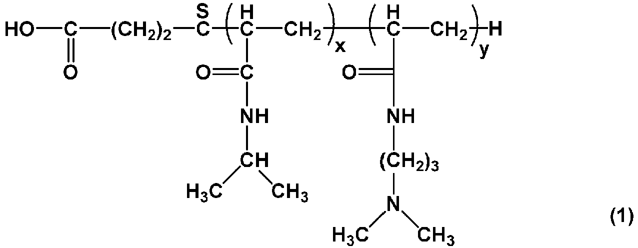

There is no particular limitation for the molecular weight of the temperature-responsive polymer for use in an embodiment of the present invention, but it may be, for example, 1000 to 100000 in terms of weight-average molecular weight. Further, the weight-average molecular weight is preferably 2000 to 50000, more preferably 3000 to 10000, and even more preferably 4000 to 7000 in view of excellent delivery of a nucleic acid into a cell and ability of more easily reducing cell death after delivery of a nucleic acid into a cell. It is noted that the weight-average molecular weight can be measured by gel filtration chromatography (GPC).

For example, in a case where a liposome is produced with a cationic lipid and a membrane fusogenic lipid, the surface of the liposome may be modified with a temperature-responsive polymer as follows. The temperature-responsive polymer is chemically modified with either the membrane fusogenic lipid or the cationic lipid. Then, the temperature-responsive polymer modified with either of the lipids is mixed when producing the liposome. As a result, a structure having a surface modified with the temperature-responsive polymer can be obtained after this liposome production.

The conventionally known method can be used for producing the liposome according to an embodiment of the present invention. For example, the liposome according to an embodiment of the present invention can be produced with a cationic lipid and a membrane fusogenic lipid as follows. The cationic lipid and the membrane fusogenic lipid are dissolved into a solvent (for example, chloroform and the like). The solvent is then removed, and the resulting thin lipid film is then hydrated with the phosphate buffered saline and the like, and then sonicated to adjust the particle size of the liposome.

There is no particular limitation for the mean particle size of the liposome, but it may be, for example, 0.05 to 1 µm. The mean particle size of the liposome is measured by the dynamic light scattering method (DLS).

There is no particular limitation for the types of cells to which the carrier for delivering a nucleic acid according to an embodiment of the present invention, but they include, for example, cells such lung cells, colon cells, rectum cells, anus cells, bile duct cells, small intestine cells, gastric cells, esophagus cells, gallbladder cells, liver cells, pancreatic cells, appendix cells, breast cells, ovarian cells, cervical cells, prostate cells, kidney cells, glial cells, skin cells, lymph cells, villous cells, cervicofacial cells, osteogenic cells, and blood cells or cancer cells thereof, (cervical cancer cells, lung cancer cells, colon cancer cells, rectum cancer cells, anal cancer cells, bile duct cancer cells, small intestine cancer cells, gastric cancer cells, esophagus cancer cells, gallbladder cancer cells, liver cancer cells, pancreatic cancer cells, appendix cancer cells, breast cancer cells, ovarian cancer cells, prostate cancer cells, kidney cancer cells, cancer cells of central nervous system, glioblastoma cells, skin cancer cells, lymphoma cells, villous cancer cells, cervicofacial cancer cells, osteogenic sarcoma cells, blood cancer cells, and the like). The carrier for delivering a nucleic acid according to an embodiment of the present invention is preferably used for delivery of a nucleic acid into a cancer cell in view of delivery efficiency of a nucleic acid into a cell and ability of more easily reducing cell death after delivery of a nucleic acid. Among cancer cells, it is particularly suitable for delivering a nucleic acid into a cervical cancer cell, a colon cancer cell, a renal cancer cells, and a lymph cell.

There is no particular limitation for the nucleic acid to be delivered, but it may be, for example, siRNA, mRNA, tRNA, rRNA, cDNA, miRNA, cyclic plasmid DNA, and the like. These nucleic acids may be any of single-stranded, double-stranded, or triple-stranded. Further, in the present invention, the nucleic acid may be chemically modified, or may be modified with an enzyme, a peptide, and the like. Moreover, the nucleic acid(s) may be used alone or in an appropriate combination of two or more. siRNA as a nucleic acid is to be preferably delivered in view of an excellent effect for reducing cell death after delivery of a nucleic acid and excellent delivery efficiency of a nucleic acid into a cell. Further, there is no particular limitation for the number of bases of a nucleic acid, but it may vary appropriately, and may be selected from, for example, the range of 10 to 10000 bp. That is, according to the carrier for delivering a nucleic acid according to an embodiment of the present invention, a short nucleic acid with the number of bases as small as 10 to 50 bp can be delivered, and even a long nucleic acid with the number of bases as large as 1000 to 10000 bp can be delivered.

### <Kit for delivering nucleic acid>

The kit for delivering a nucleic acid according to an embodiment of the present invention includes the aforementioned carrier for delivering a nucleic acid.

The kit for delivering a nucleic acid according to an embodiment of the present invention may include a component other than the aforementioned carrier for delivering a nucleic acid. For example, components of the kit for delivering a nucleic acid according to an embodiment of the present invention may include a container containing a dispersed liquid in which the aforementioned carrier for delivering a nucleic acid is dispersed in a solvent (water, buffer, sucrose, and the like), an operation manual in which instructions for using the kit are described, and the like. Moreover, those components (for example, sucrose and others) other than the carrier for delivering a nucleic acid in addition to a solvent such as water and buffer may be included in the dispersion liquid containing the carrier for delivering a nucleic acid.

### <Method of delivering nucleic acid into cell>

The method of delivering a nucleic acid according to an embodiment of the present invention includes the step of: contacting a complex of the aforementioned carrier for delivering a nucleic acid and a nucleic acid with an isolated cell, or administering the complex of the carrier for delivering a nucleic acid and a nucleic acid to a non-human animal.

### (Contact step)

The contact step in the method of delivering a nucleic acid according to an embodiment of the present invention is a step of contacting a complex of the aforementioned carrier for delivering a nucleic acid and a nucleic acid with an isolated cell. The step is performed in vitro. This enables the nucleic acid to be delivered into the cell through the aforementioned carrier for delivering a nucleic acid.

The complex of the carrier for delivering a nucleic acid and a nucleic acid may be formed by the conventionally known method. For example, it may be produced by dispersing the carrier for delivering a nucleic acid and a nucleic acid each in a separate medium (serum MEM (a minimum essential medium and others)), and mixing these.

There is no particular limitation for the molar ratio (N/P) of nitrogen and phosphorus in the liposome-nucleic acid complex, but it is in particular preferably 5:0.1 to 5:10, more preferably 5:0.5 to 5:8, and even more preferably 5:0.7 to 5:2.0 in view of excellent delivery efficiency of a nucleic acid into a cell. The molar ratio (N/P) of nitrogen and phosphorus in the liposome-nucleic acid complex can be measured by the zeta-potential in accordance with the dynamic light scattering method (DLS).

Contacting can be performed by the conventional known method. The complex of the carrier for delivering a nucleic acid and a nucleic acid is administered to a medium containing cells, and incubated at 37 to 42°C for 20 minutes to 48 hours to perform diffusion administration into a cell.

Cells to be contacted may be human or non-human cells as long as they are isolated. Further, those similar to the cell into which a nucleic acid is delivered in the carrier for delivering a nucleic acid as described above can be used.

### (Administration step)

The administration step in the method of delivering a nucleic acid according to an embodiment of the present invention is a step of administering the complex of the aforementioned carrier for delivering a nucleic acid and a nucleic acid to a non-human animal. The step is performed in vivo. This enables the nucleic acid to be delivered into a cell of a non-human animal through the aforementioned carrier for delivering a nucleic acid.

There is no particular limitation for the mode of administration, but it can be suitably selected depending on the type of target cells. For example, the mode of administration may be oral and injection (intravenous injection, subcutaneous injection, intramuscular injection, and the like).

There is no particular limitation for the non-human animal, but it may be, for example, animals such as mammals, for example, mouse, rat, canine, feline, simian, swine, bovine, sheep, rabbit, and the like.

### EXAMPLES

### <Preparation of carrier for delivering nucleic acid - 1>

### (Example 1)

### [Synthesis of temperature-responsive polymer]

NIPAAm (N-isopropylacrylamide) represented by the formula (2) and DMAPAAm (methylaminopropylacrylamide) represented by the formula (3) were dissolved in DMF (dimethylformamide) to give a molar ratio of 95:5. It is noted that the total amount of NIPAAm and DMAPAAm was 10 g. MPA (3-mercaptopropionic acid) was added to the solvent after the dissolution so as to be 0.028 times of NIPAAm in terms of the number of moles, and 58 mg of AIBN (azobisisobutyronitrile) was further added. Subsequently, the solvent was degassed by purging with nitrogen gas and sonication, and then radical polymerization was performed at 70°C for 5 hours. The liquid after the reaction was cooled back to the room temperature, and then added dropwise to pre-cooled diethyl ether to perform reprecipitation purification. Then, precipitates were collected by filtration. This reprecipitation purification was performed two more times to collect a temperature-responsive polymer represented by the formula (1). The above temperature-responsive polymer represented by the formula (1) was dialyzed against water, and then lyophilization was performed to further purify the temperature-responsive polymer represented by the formula (1). It is noted that the molecular weight of the temperature-responsive polymer represented by the formula (1) is 5500, and the lower critical solution temperature with water is 40°C. The reaction scheme is shown below.

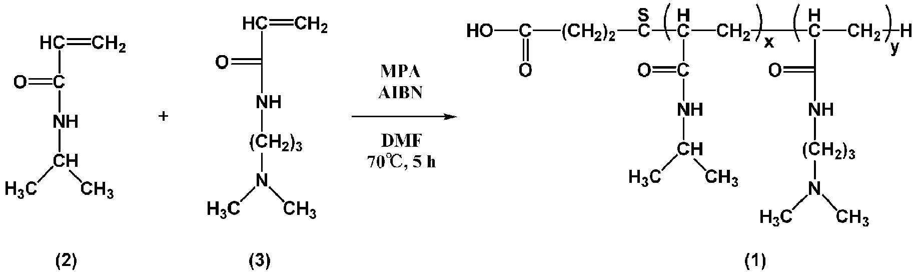

### [Synthesis of lipid-modified temperature-responsive polymer]

In order to convert the temperature-responsive polymer represented by the formula (1) to an active ester, the temperature-responsive polymer represented by the formula (1), DCC (N,N'-dicyclohexylcarbodiimide), and NHS (N-hydroxysuccinimide) were dissolved in methyl chloride in the molar ratio of 1:2.5:2.5, and then allowed to react at the room temperature for 24 hours. After the reaction, dicyclohexyl urea as a by-product was removed by suction filtration, and reprecipitation purification was then performed with diethyl ether, and a succinyl polymer represented by the formula (4) was collected as precipitates. The succinyl polymer represented by the formula (4) and a membrane fusogenic lipid DOPE (1,2-dioleoyl-sn-glycero-3-phosphoethanolamine) in the molar ratio of 1:1 were dissolved in dioxane, and allowed to react at the room temperature for 24 hours. The solvent of the solution after the reaction was evaporated under reduced pressure with an evaporator to prepare a lipid-modified temperature-responsive polymer represented by the formula (5). The reaction scheme is shown below.

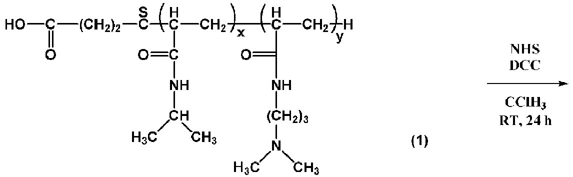

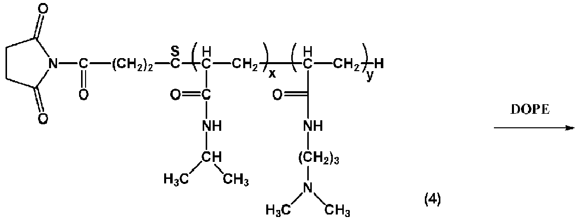

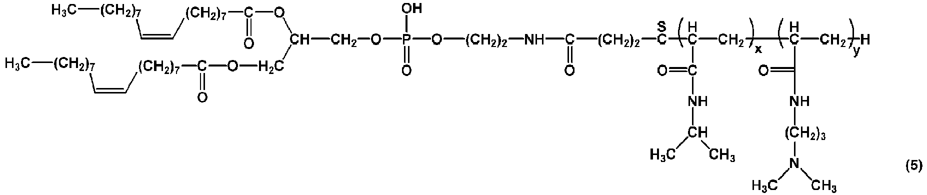

### [Production of liposome]

DOTAP (1,2-dioleoyl-3-trimethylammonium propane) as a cationic lipid and DOPE (including the lipid-modified temperature-responsive polymer represented by the formula (5)) were dissolved in chloroform so that the molar ratio was 3:7, and the total lipid amount was 20 mg. It is noted that the molar ratio of DOPE and the lipid-modified temperature-responsive polymer represented by the formula (5) was 6.5:0.5 in the DOPE solution of chloroform. The resulting solution was transferred to an eggplant flask, and the solvent was evaporated in an evaporator to form a lipid film. To the lipid film, 1 ml of purified water was added to hydrate the lipid film, and then dispersed by vortex. Then, sonication was performed for 30 minutes in an ultrasonic bath to reduce the size of the liposome, and then passed through a 100-nm filter with an extruder to adjust the size of the liposome. Then, gel filtration was performed to separate and purify the liposome to obtain the liposome of interest according to Example 1 having a surface modified with the temperature-responsive polymer represented by the formula (1).

### (Comparative Example 1)

Lipofectamine® available from Invitrogen Inc. (Lipofectamine® RNAi MAX®) was provided, and used as a liposome according to Comparative Example 1.

### (Comparative Example 2)

A liposome according to Comparative Example 2 was produced by a similar procedure as in Example 1 except that liposome production was performed without including the lipid-modified temperature-responsive polymer represented with the formula (5) in DOPE. The liposome according to Comparative Example 2 is similar to the liposome according to Example 1 except that the surface is not modified with the temperature-responsive polymer represented by the formula (1).

### (Comparative Example 3)

A liposome according to Comparative Example 3 was produced by a similar procedure as in Example 1 except that PEG (polyethylene glycol, the molecular weight: 2000) was used instead of the temperature-responsive polymer represented by the formula (1).

### <Measurement of luciferase activity after delivery of siRNA into cells - 1>

A dispersed liquid of a complex with a nucleic acid was prepared for each of the liposomes according to Example 1 and Comparative Examples 1 to 3, and this dispersion liquid was used to delivery the nucleic acid to HeLa cells. siRNA (siRNA duplex available from Sigma Genesis) which suppress the luciferase activity was used as the nucleic acid. Below, shown is the amount of a liposome and siRNA in the dispersed liquid of the complex and siRNA for each of Example 1 and Comparative Examples 1 to 3. It is noted that the final concentration of siRNA in the dispersion liquid of the complex and siRNA was 50 nM for all. Example 1: liposome-siRNA complex/siRNA (N/P (the molar ratio of nitrogen and phosphorus)) = 5:1
Comparative Example 1: siRNA:liposome = 0.1 nmol:10 µl (Lipofectamine® available from Invitrogen Inc. (dispersed in a liquid) 10 µl)
Comparative Example 2: liposome-siRNA complex/siRNA (N/P) = 5:1
Comparative Example 3: liposome-siRNA complex/siRNA (N/P) = 5:1

Specifically, a dispersed liquid of the complex with a nucleic acid was produced, and the nucleic acid was delivered into cells according to the following method. First, siRNA was mixed with non-FBS (non-fetal bovine serum) (serum MEM (minimum essential medium)) in each well of a 6-well plate so that the final concentration was 50 nM, and the total volume of 125 µl, and allowed to stand for 5 minutes. Meanwhile, the liposomes from Example 1 and Comparative Examples 1 to 3 were each dispersed in 125 µl of non-FBS medium (serum MEM (minimum essential medium)), and allowed to stand for 5 minutes. These two non-FBS media were mixed to prepare a dispersed liquid of the liposome-siRNA complex, and allowed to stand for 30 minutes. Then, FBS medium was added to the dispersed liquid to a volume of 1 ml. After the volume adjustment, a medium of HeLa cells (cervical cancer cells) seeded in a 6-well plate at the concentration of 5.0x10⁴ cells/ml on the previous day was replaced with the dispersion liquid of the liposome-siRNA complex, and incubated at 37°C for 48 hours. After the incubation, the medium was removed from each well, and 250 µl/well of a cell-lysis agent (a Pikkagene cell-lysis agent Lcβ (Toyo Ink, Co., Ltd.)) diluted 5 times with PBS was added. After incubated under the above conditions for 30 minutes, the cells were detached by pipetting, and the resulting solution was transferred to a micro tube. Then, the micro tube was frozen at -80°C to collapse the cells. After collapsing the cells, the solution was thawed in a 37°C bath, and then centrifuged under conditions of 12000 rpm and 4°C for 5 minutes to spin down impurites. Of the resulting supernatant, 90 µl was transferred to another micro tube, from which 10 µl was added to a 96-well plate. To this, 50 µl of a Pikkagene solution was further added, and luminescence measurements were performed to determine the luciferase activity after delivery of siRNA into HeLa cells. The luciferase activity was evaluated for the unit emission amount for each. The unit emission amount (C/P) was defined as a quotient of a detected emission amount, which is denoted as C (count per second), divided by protein abundance P (protein abundant). It is noted that the luciferase activities for Example 1 and Comparative Examples 1 to 3 were evaluated in a relative manner as follows. In addition to the above materials, 10 µl of a medium of HeLa cells into which siRNA was not delivered was added to a 96-well plate. A BCA (bicinchoninic acid) assay kit was used to create a calibration curve with BCA standards having concentrations in the range of 0 to 1 mg/ml, and the amount of proteins was then determined. The emission amount C and the resulting protein abundance P were used to calculate the unit emission amount (C/P), which was assumed as 100% (control).

### (Control Example 1)

The luciferase activity was measured in similar conditions as described above for the liposome from Example 1 except that the complex with siRNA was not formed, and the liposome alone was delivered into the cells.

### (Control Example 2)

The luciferase activity was measured in similar conditions as described above for the liposome from Comparative Example 1 except that the complex with siRNA was not formed, and the liposome alone was delivered into the cells.

### <Measurement of cell viability after delivery of siRNA into cells>

siRNA was mixed with non-FBS (non-fetal bovine serum) (serum MEM (minimum essential medium)) in each well of a 96-well plate so that the final concentration was 100 nM, and the total volume was 125 µl, and allowed to stand for 5 minutes. Meanwhile, the liposomes from Example 1 and Comparative Example 1 were each dispersed in 125 µl of non-FBS medium (serum MEM (minimum essential medium)), and allowed to stand for 5 minutes. These two non-FBS media were mixed to prepare a dispersion liquid of the liposome-siRNA complex, and allowed to stand for 30 minutes. Then, FBS medium was added to the dispersed liquid to a volume of 1 ml. After the volume adjustment, a medium of HeLa cells (cervical cancer cells) seeded in a 6-well plate at the concentration of 5.0x10⁴ cells/ml on the previous day was replaced with the dispersion liquid of the liposome-siRNA complex, and incubated at 37°C for 48 hours. After the incubation, 10 µl of a WST-8 solution (Cell Counting Kit-8, Dojindo Molecular Technologies, Inc.) was added to each well, and further incubated at 37°C for 30 minutes, and then the absorbance at 450 nm was measured to determine cell viability. Moreover, untreated HeLa cells was measured at 450 nm, and the obtained numerical value was assumed to be 100% cell viability (control), and used for relative evaluation of cell viability for Example 1 and Comparative Example 1.

### (Control Example 3)

The cell viability was measured in a similar way as described above for the liposome from Example 1 except that the complex with siRNA was not formed, and the liposome alone was delivered into the cells.

### (Control Example 4)

The cell viability was measured in a similar way as described above for the liposome from Comparative Example 1 except that the complex with siRNA was not formed, and the liposome alone was delivered into the cells.

### <Evaluation results>

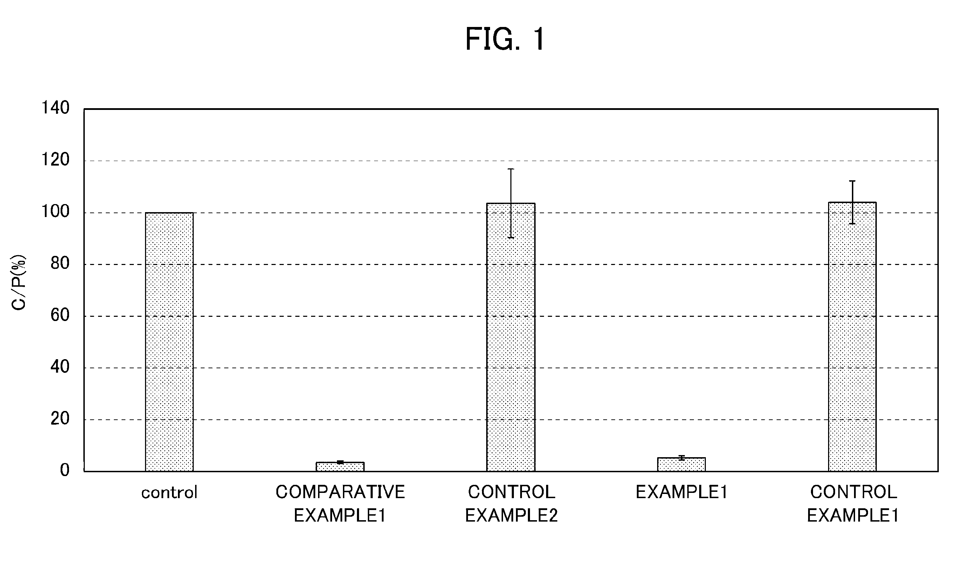

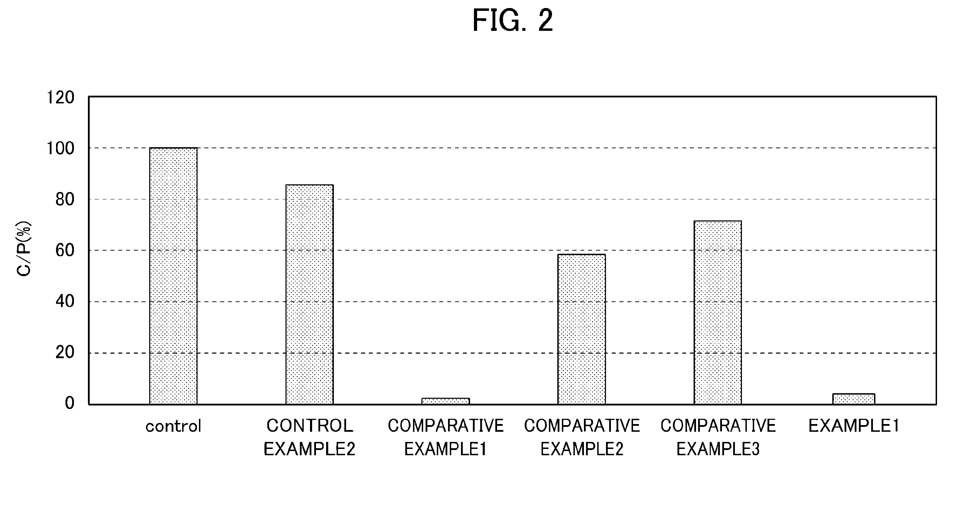

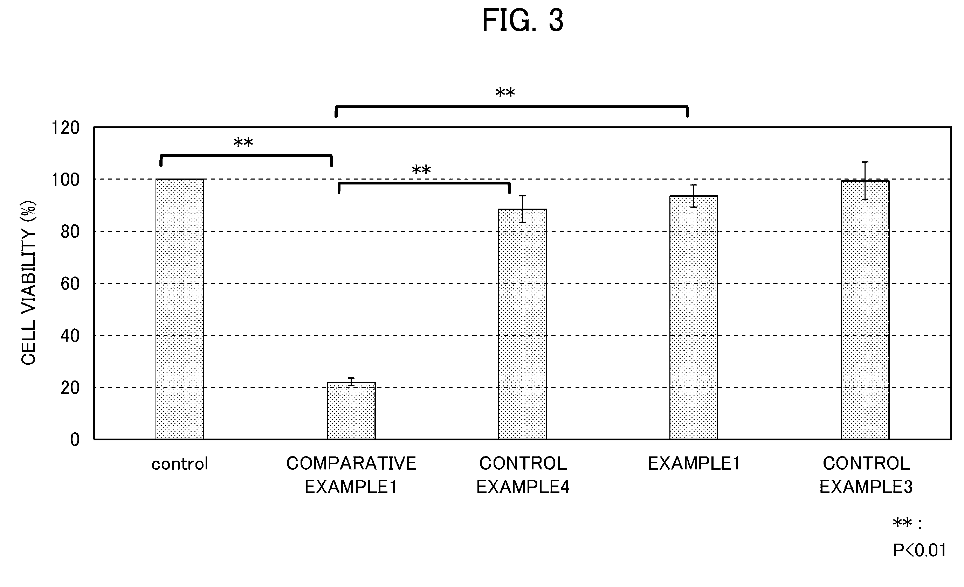

Fig. 1 shows measurement results of the luciferase activity for Example 1, Comparative Example 1, and Control Examples 1 and 2. Fig. 2 shows measurement results of the luciferase activity for Example 1, Comparative Examples 1 to 3, and Control Example 2. Fig. 3 shows measurement results of the cell viability for Example 1, Comparative Example 1, and Control Examples 3 and 4.

The graph shown in Fig. 1 revealed that the luciferase activity from Comparative Example 1 where Lipofectamine® was used to deliver siRNA was similar to the luciferase activity from Example 1 where a liposome having a surface modified with the temperature-responsive polymer represented by the formula (1) was used to deliver siRNA. Further, the graph shown in Fig. 2 revealed that the luciferase activity from Example 1 was higher than that from Comparative Example 2 (the luciferase activity when a liposome having a similar configuration as that of Example 1 except that the surface was not modified with the temperature-responsive polymer represented by the formula (1)), and that from Comparative Example 3 (the luciferase activity when the same liposome as that from Example 1 except that the surface was modified with PEG instead of the temperature-responsive polymer represented by the formula (1)). These results showed that the liposome having a surface modified with the temperature-responsive polymer had superior delivery efficiency of a nucleic acid into a cell to the liposome having a surface modified with PEG and the liposome having an unmodified surface, and had a similar delivery efficiency of a nucleic acid into a cell to that of the conventional Lipofectamine®.

As seen in the graph of Fig. 3, the cell viability of Example 1 is significantly better than that of Comparative Example 1. In addition to that, the cell viability of Example 1 was found to be similar that of untreated HeLa cells (control). As understood from the above cell viability, Example 1 showed a superior effect for reducing cell death after delivery of a nucleic acid to the conventional Lipofectamine®.

These results as described above indicated that the carrier for delivering a nucleic acid according to an embodiment of the present invention had an excellent delivery efficiency of a nucleic acid into a cell, and further had an excellent effect for reducing cell death after delivery of a nucleic acid regardless of inclusion of a cationic lipid as a membrane constituent. The reason of this may be explained as follows. The surface of the liposome of the carrier for delivering a nucleic acid according to an embodiment of the present invention is modified with a temperature-responsive polymer. Therefore, a hydration layer is formed at the lower critical solution temperature, which can reduce cytotoxicity due to a cationic lipid. On the other hand, when the polymer becomes hydrophobic at an elevated temperature, the hydration layer is decreased, leading to improved cell affinity. This enables direct delivery of a nucleic acid into a cell without via a lysosome by virtue of the lipid having membrane fusion capability.

### <Measurement of luciferase activity after delivery of siRNA into cells - 2>

For each of the liposomes from Example 1, Comparative Example 1, and Comparative Example 3, the dispersion liquid of the complex with a nucleic acid was produced, which was then used to deliver the nucleic acid to HeLa cells under different temperature conditions. siRNA (siRNA duplex available from Sigma Genesis) which suppresses the luciferase activity was used as the nucleic acid. Below, shown is the amount of a liposome and siRNA in the dispersion liquid of the complex with siRNA for Example 1 and Comparative Example 1. It is noted that the final concentration of siRNA in the dispersion liquid of the complex and siRNA was 50 nM for all. Example 1: liposome-siRNA complex / siRNA (N/P (the molar ratio of nitrogen and phosphorus)) = 5:1
Comparative Example 1: siRNA:liposome = 0.1 nmol:10 µl (Lipofectamine® available from Invitrogen Inc. (dispersed in a liquid) 10 µl)
Comparative Example 3: liposome-siRNA complex/siRNA (N/P) = 5:1

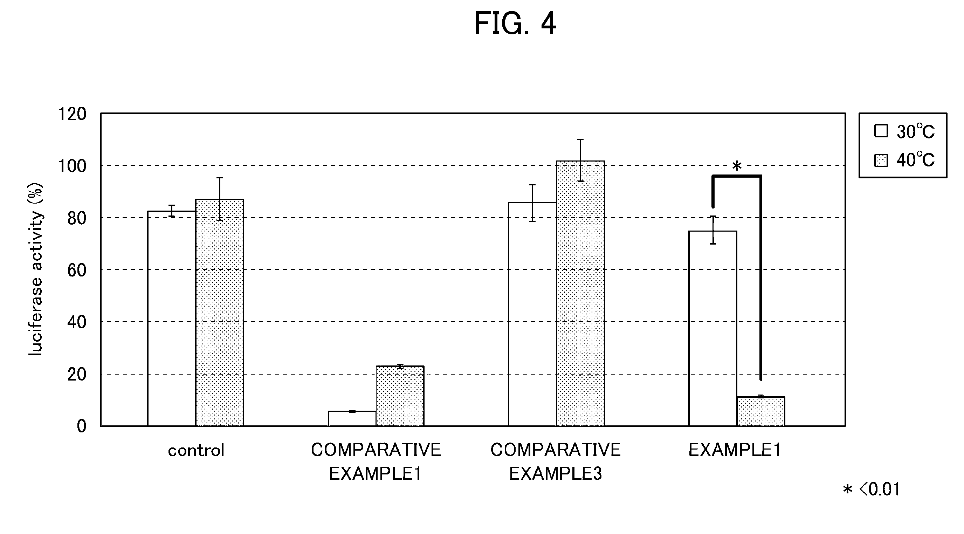

Specifically, the dispersed liquid of the complex with a nucleic acid was produced, and the nucleic acid was delivered into cells according to the following method. First, siRNA was mixed with non-FBS (non-fetal bovine serum) (serum MEM (minimum essential medium)) in each well of a 6-well plate so that the final concentration was 50 nM, and the total volume of 125 µl, and allowed to stand for 5 minutes. Meanwhile, the liposomes from Example 1 and Comparative Examples were each dispersed in 125 µl of non-FBS medium (serum MEM (minimum essential medium)), and allowed to stand for 5 minutes. These two non-FBS media were mixed to prepare a dispersion liquid of the liposome-siRNA complex, and allowed to stand for 30 minutes. Then, FBS medium was added to the dispersed liquid to a volume of 1 ml. After the volume adjustment, a medium of HeLa cells (cervical cancer cells) seeded in a 6-well plate at the concentration of 1.0x10⁵ cells/ml on the previous day was replaced with the dispersion liquid of the liposome-siRNA complex, and incubated at 30°C or 40°C for 48 hours. After the incubation, the medium was removed from each well, and 250 µl/well of a cell-lysis agent (a Pikkagene cell-lysis agent Lcβ (Toyo Ink, Co., Ltd.)) diluted 5 times with PBS was added. After incubated under the above conditions for 30 minutes, the cells were detached by pipetting, and the resulting solution was transferred to a micro tube. Then, the micro tube was frozen at -80°C to collapse the cells. After collapsing the cells, the solution was thawed in a 37°C bath, and then centrifuged under conditions of 12000 rpm and 4°C for 5 minutes to spin down impurites. Of the resulting supernatant, 90 µl was transferred to another micro tube, from which 10 µl was added to a 96-well plate. To this, 50 µl of a Pikkagene solution was further added, and luminescence measurements were performed to determine the luciferase activity after delivery of siRNA into HeLa cells. The luciferase activity was evaluated for the unit emission amount for each. The unit emission amount (C/P) was defined as a quotient of a detected emission amount denoted as C (count per second) divided by protein abundance P (Protein abundant). It is noted that the luciferase activities for Example 1 and Comparative Examples 1 and 3 were evaluated in a relative manner as follows. In addition to the above materials, 10 µl of a medium of HeLa cells into which siRNA was not delivered was added to a 96-well plate. A BCA (bicinchoninic acid) assay kit was used to create a calibration curve with BCA standards having concentrations in the range of 0 to 1 mg/ml, and the amount of proteins was then determined. The emission amount C and the resulting protein abundance P were used to calculate the unit emission amount (C/P), which was assumed as 100% (control). The results are shown in Fig. 4.

As seen from the graph of Fig. 4, the liposome modified with the temperature-responsive polymer did not sufficiently suppress the luciferase activity at a temperature at or below the lower critical solution temperature (30°C) whilst it suppressed the luciferase activity as in the conventional Lipofectamine® at a temperature at or above the lower critical solution temperature (40°C).

The above results showed that the liposome having a surface modified with the temperature-responsive polymer was able to control the delivery of a nucleic acid into a cell by adjusting the temperature at or above or at or below the lower critical solution temperature.

### <Preparation of carrier for delivering nucleic acid - 2>

### (Example 2)

### [Synthesis of temperature-responsive polymer]

NIPAAm (N-isopropylacrylamide) represented by the formula (2) and DMAAm (dimethylaminoacrylamide) represented by the formula (7) was dissolved in DMF (dimethylformamide) to give a molar ratio of 69.5:30.5. It is noted that the total amount of NIPAAm and DMAAm was 10 g. MPA (3-mercaptopropionic acid) was added to the solvent after the dissolution so as to be 0.028 times of NIPAAm in terms of the number of moles, and 58 mg of AIBN (azobisisobutyronitrile) was further added. Subsequently, the solvent was degassed by purging with nitrogen gas and sonication, and then radical polymerization was performed at 70°C for 5 hours. The liquid after the reaction was cooled back to the room temperature, and then added dropwise to pre-cooled diethyl ether to perform reprecipitation purification. Then, precipitates were collected by filtration. This reprecipitation purification was performed two more times to collect a temperature-responsive polymer represented by the formula (6). The above temperature-responsive polymer represented by the formula (6) was dialyzed against water, and then lyophilization was performed to further purifying the temperature-responsive polymer represented by the formula (6). It is noted that the molecular weight of the temperature-responsive polymer represented by the formula (6) is 5000, and the lower critical solution temperature is 41.8°C with water. The reaction scheme is shown below.

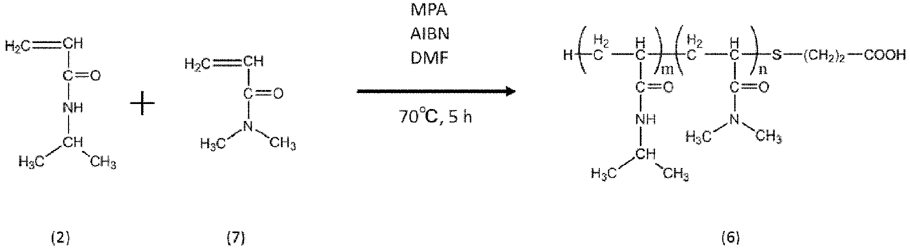

### [Synthesis of lipid-modified temperature-responsive polymer]

In order to convert the temperature-responsive polymer represented by the formula (6) to an active ester, the temperature-responsive polymer represented by the formula (6), DCC (N,N'-dicyclohexylcarbodiimide), and NHS (N-hydroxysuccinimide) were dissolved in chloroform to give a molar ratio of 1:2.5:2.5, and DOPE (1,2-dioleoyl-sn-glycero-3-phosphoethanolamine) as a membrane fusogenic lipid was further added so that the molar ratio to the polymer was 1:1, and then allowed to react at the room temperature for 24 hours. The solution after the reaction was dialyzed against a methanol solution to purify the product. The solvent was evaporated under reduced pressure with an evaporator to prepare a lipid-modified temperature-responsive polymer represented by the formula (8). The reaction scheme is shown below.

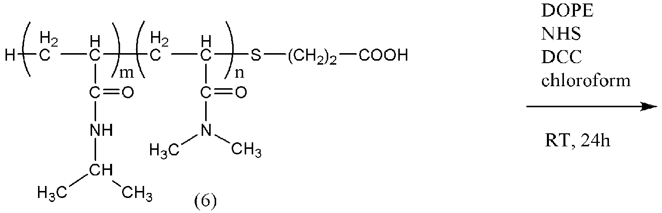

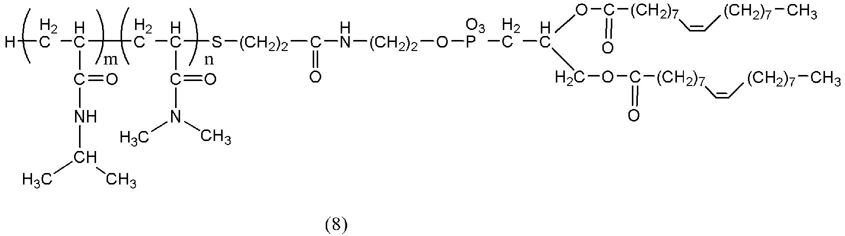

### [Production of liposome]

DOTAP (1,2-dioleoyl-3-trimethylammonium propane) as a cationic lipid and DOPE (including the lipid-modified temperature-responsive polymer represented by the formula (8)) were each dissolved in chloroform so that the molar ratio was 3:7, and the total lipid amount was 20 mg. It is noted that the molar ratio of DOPE and the lipid-modified temperature-responsive polymer represented by the formula (8) was 6.5:0.5 in the DOPE solution of chloroform. The resulting solution was transferred to an eggplant flask, and the solvent was evaporated in an evaporator to form a lipid film. To the lipid film, 1 ml of purified water was added to hydrate the lipid film, and then dispersed by vortex. Then, sonication was performed for 30 minutes in an ultrasonic bath to reduce the size of the liposome, and then passed through a 100-nm filter with an extruder to adjust the size of the liposome. Then, gel filtration was performed to separate and purify the liposome to obtain the liposome of interest according to Example 2 having a surface modified with the temperature-responsive polymer represented by the formula 6.

### <Measurement of luciferase activity after delivery of siRNA into cells - 1>

The liposome from Example 2 was used to produce a dispersion liquid of a complex with a nucleic acid, which was used to deliver the nucleic acid to HeLa cells under different temperature conditions. siRNA (siRNA duplex available from Sigma Genesis) which suppresses the luciferase activity was used as the nucleic acid. Below, shown is the amount of a liposome and siRNA in the dispersion liquid of the complex with siRNA for Example 2. It is noted that the final concentration of siRNA in the dispersion liquid of the complex and siRNA was 50 nM for all.

### Example 2 liposome-siRNA complex / siRNA (N/P (the molar ratio of nitrogen and phosphorus)) = 5:1

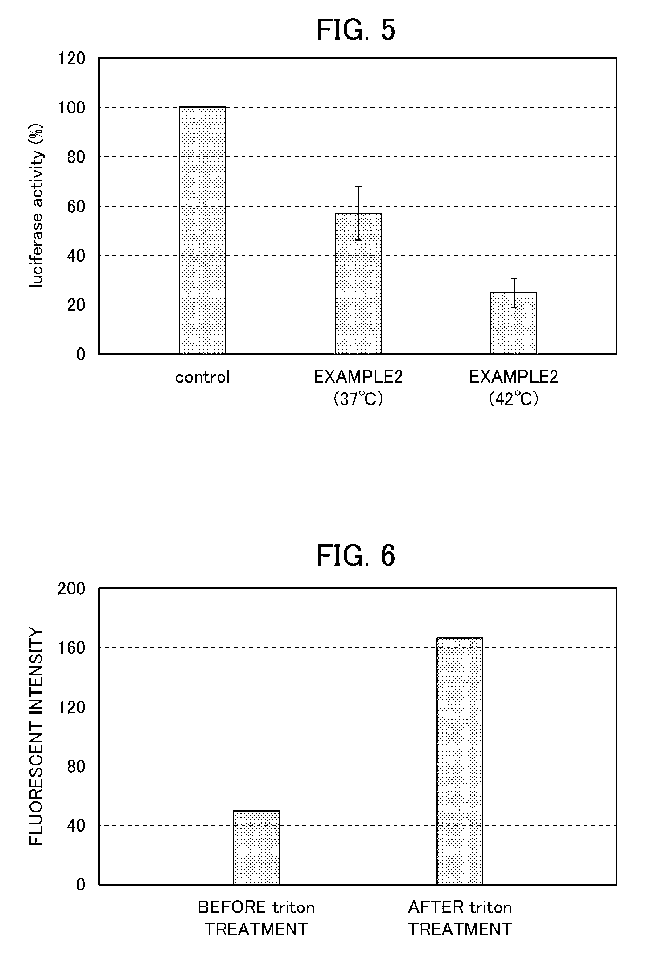

Specifically, the dispersed liquid of the complex with a nucleic acid was produced, and the nucleic acid was delivered into cells according to the following method. First, the siRNA was mixed with non-FBS (non-fetal bovine serum) (serum MEM (minimum essential medium)) in each well of a 6-well plate so that the final concentration was 50 nM, and the total volume was 125 µl, and allowed to stand for 5 minutes. Meanwhile, each liposome from Example 2 was dispersed in 125 µl of non FBS medium (serum MEM (minimum essential medium)), and allowed to stand for 5 minutes. These two non-FBS media were mixed to prepare a dispersion liquid of the liposome-siRNA complex, and allowed to stand for 30 minutes. Then, FBS medium was added to the dispersed liquid to a volume of 1 ml. After the volume adjustment, a medium of HeLa cells (cervical cancer cells) seeded in a 6-well plate at the concentration of 5.0x10⁴ cells/ml on the previous day was replaced with the dispersion liquid of the liposome-siRNA complex, and incubated at 37°C or 42°C for 48 hours. After the incubation, the medium was removed from each well, and 250 µl/well of a cell-lysis agent (a Pikkagene cell-lysis agent Lcβ (Toyo Ink, Co., Ltd.)) diluted 5 times with PBS was added. After incubated under the above conditions for 30 minutes, the cells were detached by pipetting, and the resulting solution was transferred to a micro tube. Then, the micro tube was frozen at -80°C to collapse the cells. After collapsing the cells, the solution was thawed in a 37°C bath, and then centrifuged under conditions of 12000 rpm and 4°C for 5 minutes to spin down impurites. Of the resulting supernatant, 90 µl was transferred to another micro tube, from which 10 µl was added to a 96-well plate. To this, 50 µl of a Pikkagene solution was further added, and luminescence measurements were performed to determine the luciferase activity after delivery of siRNA into HeLa cells. The luciferase activity was evaluated for the unit emission amount for each. The unit emission amount (C/P) was defined as a quotient of a detected emission amount denoted as C (count per second) divided by protein abundance P (Protein abundant). It is noted that the luciferase activity for Example 2 were evaluated in a relative manner as follows. In addition to the above materials, 10 µl of a medium of HeLa cells into which siRNA was not delivered was added to a 96-well plate. A BCA (bicinchoninic acid) assay kit was used to create a calibration curve with BCA standards having concentrations in the range of 0 to 1 mg/ml, and the amount of proteins was then determined. The emission amount C and the resulting protein abundance P were used to calculate the unit emission amount (C/P), which was assumed as 100% (control). The results are shown in Fig. 5.

As seen from the results in Fig. 5, the siRNA transfection with the liposome from Example 2 in which the temperature-responsive polymer having a different composition from that of Example 1 was also found to suppress the luciferase activity, showing a stronger suppression of the activity at or above LCST (the lower critical solution temperature) as compared with that at or below LCST (the lower critical solution temperature).

### <Measurement of encapsulation amount of carboxyfluorescein>

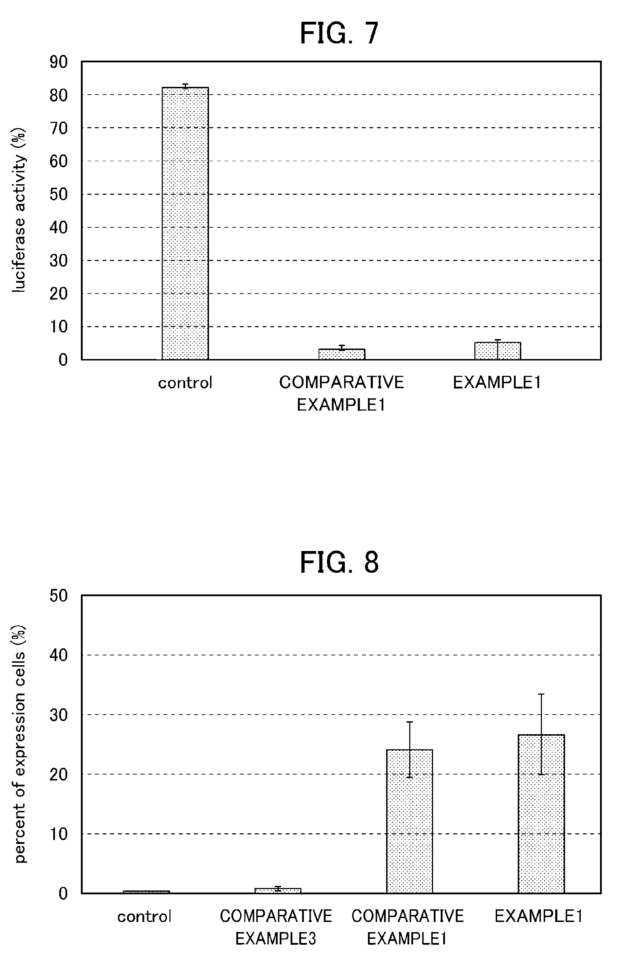

A liposome according to Example 1 was prepared, and 1 ml of PBS containing 2 mM carboxyfluorescein (CF) instead of purified water was added to the lipid film for hydration. The resulting liposome was diluted 20 times with PBS, and measured for a fluorescence intensity with a fluorophotometer, which was considered as the fluorescence intensity at the time of CF encapsulation. Further, 1% Triton 10X was added to completely collapse the liposome, and then the fluorescence intensity was measured, which was considered as the fluorescence intensity upon complete release. The value of the fluorescence intensity upon encapsulation was subtracted from the value of the fluorescence intensity upon addition of Triton to calculate the difference, which was considered as the encapsulation amount. The fluorescence intensities before and after Triton treatment are shown in Fig. 6. The fluorescence intensity increased after Triton treatment as shown in Fig. 6, showing that carboxyfluorescein was able to be encapsulated.

### <siRNA transfection with CF-encapsulated liposome>

The liposome from Example 1 which was encapsulated with carboxyfluorescein and the liposome (Lipofectamine®) from Comparative Example 1 were measured for the luciferase activity to determine whether they had siRNA transfection capability. The results are shown in Fig. 7. As shown in Fig. 7, the luciferase activity was decreased in a similar way as in Comparative Example 1, indicating that Example 1 had a similarly efficient transfection capability. That is, the liposome from Example 1 was able to encapsulate a low-molecular material carboxyfluorescein therein, and was able to be used to produce a complex with siRNA, and was able to show a transfection capability as efficient as Lipofectamine®.

### <Plasmid transfection>

The liposome from Comparative Example 1 (Lipofectamine® (Lipofectamine® 2000), Invitrogen Inc.) and the liposome from Comparative Example 3 (modified with PEG instead of a temperature-responsive polymer) were prepared. Further, the liposome from Example 1 was prepared. The liposomes from Example 1, Comparative Examples 1 and 2 were each used to produce a dispersion liquid of a complex with a nucleic acid (plasmid), which was then used to deliver the nucleic acid (plasmid) into HeLa cells. Specifically, the dispersed liquid of the complex with the nucleic acid was produced, and the nucleic acid was delivered into cells according to the following method.

A MSCV-IRES-GFP plasmid in an amount of 5 µg was dispersed in 50 µl of serum-free medium. Further, 3 µl of Lipofectamine® 2000 and 2 µl of each liposome were dispersed in 50 µl of serum-free medium. These two media were mixed, and allowed to stand for 30 minutes to produce a liposome-plasmid complex. Then, a serum-containing medium was added to the dispersed liquid of the complex to a volume of 1 ml. After the volume adjustment, a medium of HeLa cells seeded in each well of a 6-well plate at the concentration of 5.0x10⁴ cells/ml on the previous day was replaced with the medium in which the liposome-plasmid complex was dispersed, and incubated at 40°C for 48 hours. After the incubation, the medium was removed, and trypsin treatment was performed to strip the cells from the dish. PBS was then added to the cells, and centrifugation was performed to wash the cells. Subsequently, the cells were observed by flow cytometry to evaluate the expression percentage of the GFP protein in the HeLa cells. The results are shown in Fig. 8.

As seen from the results in Fig. 8, the liposome (Example 1) modified with the temperature-responsive polymer enabled significantly more efficient transfection of HeLa cells with the plasmid as compared with the PEG-liposome (Comparative Example 3), and the transfection capability thereof was comparable to or more than that of Lipofectamine®.

## Claims

1. A carrier for delivering a nucleic acid into a cell, comprising a liposome having a membrane fusion capability, wherein
the liposome includes a cationic lipid as a membrane constituent, and
a surface of the liposome is modified with a temperature-responsive polymer.

2. The carrier for delivering a nucleic acid according to claim 1, wherein the temperature-responsive polymer has a lower critical solution temperature of 35.0 to 45.0°C with water.

3. The carrier for delivering a nucleic acid according to claim 1 or 2, wherein the liposome includes a phospholipid as a membrane constituent.

4. The carrier for delivering a nucleic acid according to any one of claims 1 to 3, which is used for delivering the nucleic acid into a cervical cancer cell.

5. A kit for delivering a nucleic acid into a cell, comprising the carrier for delivering a nucleic acid according to any one of claims 1 to 4.

6. A method of delivering a nucleic acid into a cell, the method comprising the step of: contacting a complex of the carrier for delivering a nucleic acid according to any one of claims 1 to 4 and a nucleic acid with an isolated cell, or
administering the complex of the carrier for delivering a nucleic acid and a nucleic acid to a non-human animal.
